# EUROPEAN PATENT SPECIFICATION

(11) **EP 2 702 414 B1**
(45) Date of publication and mention of the grant of the patent: **08.07.2015**
(21) Application number: 12720741.3
(22) Date of filing: 26.04.2012
(51) Int. Cl.: G01N 33/96

(54) **HEMATOLOGY CONTROL COMPOSITIONS WITH EXTENDED STABILITY**
HÄMATOLOGISCHE KONTROLLZUSAMMENSETZUNGEN MIT ERHÖHTER STABILITÄT
COMPOSITIONS DE CONTRÔLE D'HÉMATOLOGIE AVEC STABILITÉ PROLONGÉE

(30) Priority: 28.04.2011 US 201161480250 P
(43) Date of publication of application: 05.03.2014
(73) Proprietor: Beckman Coulter, Inc., Brea, CA 92821 (US)
(72) Inventor: DE, Dibyendu, Pembroke Pines FL 33028 (US); PARRA-DIAZ, Dennisse, Miami FL 33183 (US); CHIN, Bruce, Miami FL 33176 (US); BAGIOTTI-SHELDON, Cristina, Pembroke Pines FL 33024 (US)
(74) Representative: Boult Wade Tennant
(86) International application number: PCT/US2012/035287
(87) International publication number: WO 2012/149211

(56) References cited:
- US-A- 5 512 485
- US-B1- 6 187 590
- NIVET C ET AL: "Plasmodium falciparum: One-step growth in a semi-defined medium and the stimulatory effect of human seric lipoproteins and liposomes", EXPERIMENTAL PARASITOLOGY, NEW YORK, NY, US, vol. 55, no. 1, 1 February 1983 (1983-02-01), pages 147-151, XP026217022, ISSN: 0014-4894, DOI: 10.1016/0014-4894(83)90008-5 [retrieved on 1983-02-01]
- WILLET G P ET AL: "Plasmodium falciparum: Continuous cultivation of erythrocyte stages in plasma-free culture medium", EXPERIMENTAL PARASITOLOGY, NEW YORK, NY, US, vol. 57, no. 1, 1 February 1984 (1984-02-01), pages 76-80, XP026445268, ISSN: 0014-4894 [retrieved on 1984-02-01]
- DATABASE BIOSIS [Online] BIOSCIENCES INFORMATION SERVICE, PHILADELPHIA, PA, US; 1991, ZHANG Z J ET AL: "METABOLISM OF N-3 POLYUNSATURATED FATTY ACIDS BY THE ISOLATED PERFUSED RAT LIVER", XP002677591, Database accession no. PREV199192073367 & LIPIDS, vol. 26, no. 7, 1991, pages 504-511, ISSN: 0024-4201

## Description

### FIELD

This present specification relates to novel suspension media for hematological compositions and methods for their use, having particular utility for compositions containing a red blood cell component and for use with devices using electronic and optical means for blood determinations.

### BACKGROUND

Hematology control products must accurately indicate, on a comparative basis, what a test sample of fresh blood constitutes with regard to the determinations in question. It is important for the control product to simulate fresh blood, since blood components, such as red blood cells, can hemolyze slowly and undergo changes in size and shape within hours after removal from a blood donor. Similarly, white blood cells suffer degenerative changes. Control suspension media should not degrade the components in the hematology control composition during the shelf life of the hematology control product. The shelf life of the hematology control product takes into account the time from manufacture of the product through the time of customer usage of the product. US5512485 describes a hematology control product comprising leukocyte analogs. The analogs are described as comprising red blood cells which simulate at least two physical properties of human leukocytes. A method is described to use the control product to determine whether an automatic instrument is operating within the manufacturer's specification.

Suspending medium formulations are designed for the purpose of stabilizing the attributes of the cells and/or particles suspended in them, and to have antimicrobial properties sufficient to inhibit the growth of possible contaminating microorganisms. The specific parameters of the red and white blood cells that are desirable to be measured dictate some of the necessary characteristics of a suitable suspension media for a hematology control product.

It is desirable to know the volume of red blood cells in a hematological sample. Once this measurement is ascertained and the red blood cells have been counted, the packed cell volume or hematocrit can be computed. Therefore, the suspension media of the control product should be capable of equilibrating and stabilizing the volume of red blood cells in the sample so that the mean corpuscular volume (MCV) and red blood cell distribution width (RDW) can be measured.

Because of the sensitivity of the components of a hematology control product, such as the red blood cell component, white blood cell component, and platelet component, to the chemical compounds contained in suspension media, it has been difficult to find a combination of chemical compounds that do not degrade or undesirably alter the characteristics of the blood cell components to be analyzed, especially over varied storage times. Frequently, one or more chemical compounds are added to maintain the stability or functional property of a blood cell component, but which might detrimentally affect one or more physical parameters of the blood cells or other blood cell determinations.

One disadvantage of prior art suspension media of hematology control products is that when used in conjunction with a red blood cell component, the control product has a short closed vial storage life. After this storage life expires, the control product does not accurately simulate one or more of the specific parameters of the blood cells that are desirable to be measured.

Historically the effectiveness of suspending media formulations has been measured by the amount of stabilization (amount of change) observed for those specific attributes of cells and/or particles that are known to change or exhibit defined trends. It is well known that mammalian red blood cells (RBCs) used in the formulation of "whole blood" hematology controls exhibit changes in their MCV and RDW over time. It is important for the red blood cell component in the hematology control product to maintain an MCV within a defined range over a sufficient period of time in order to adequately assist in the calibration, operation, and accumulation of quality assurance data for a multi-parameter automated hematology instrument. Concomitantly, the RDW should remain consistent over a sufficient period of time to provide the necessary stability for the hematology control product.

One general approach has been used to confer some degree of stability, that is, to reduce the amount of MCV change exhibited by the red blood cells utilized in "whole blood" controls. Such an approach was shown by Hunt (U.S. Pat. No. 3,873,467 (1975)) and Wong et al. (U.S. Pat. No. 4,777,139 (1988) and U.S. Pat. No. 4,698,312 (1987)), who used small amounts of various aqueous aldehydes to stabilize the shape and size of suspended red blood cells. However, one of the disadvantages of the stabilized red blood cell preparations described by Wong et al. (U.S. Pat. No. 4,777,139 (1988)) is that the stabilization period required before the exhibited MCV changes become minimal is dependent on the initial level or size of the treated preparation. Equilibrations took approximately 35, 25, and 10 days for Levels I, II, and III respectively. Once the MCV values stopped changing, they remained constant (within tolerances of 1 MCV unit) for 90 days or longer. However, the unequal equilibration times increase the logistical difficulty in implementing these teachings in a manufacturing setting. Additionally, if the red blood cells are fixed or stabilized with an aldehyde fixative, it is presently believed that the beneficial effects of the suspension media to provide long term shelf life stability is minimized, since the red blood cells will have a marked decrease in metabolic activity.

Another disadvantage of the prior art media is that, when used in conjunction with red blood cells and fixed human white blood cells or white blood cell analogs that have been stored in a closed vial for an extended period of time, the control product does not accurately indicate, on a comparative basis, what a test sample of fresh blood constitutes with regard to the determinations in question.

Thus, there exists a need in the art for new suspension media for use in hematology control products such that a stored closed vial of the hematology control product accurately indicates upon analysis, on a comparative basis, what a test sample of fresh blood constitutes with regard to the determinations of MCV and RDW. An increased shelf life avoids the problems associated with manufacturing logistics and customer dissatisfaction of obtaining products with a shortened time in which the products can be used.

The present specification meets the need in the art by providing novel suspension media with longer stability for hematology cell controls.

### SUMMARY

Aspects of the present specification provide a hematology control composition comprising a red blood cell component and an isotonic suspension medium including a serum albumin component and a cholesterol component wherein the composition has a free fatty acid concentration of less than about 4 mmol/liter and wherein the serum albumin component is present at a concentration of from 30 grams/liter to 50 grams/liter; and wherein the serum albumin component comprises a ratio of serum albumin monomer to serum albumin dimer lower than 6:1; and wherein the cholesterol component is present at a concentration of from 400 mg/liter to 1200 mg/liter.

Yet other aspects of the present specification provide a method to determine operational accuracy and reproducibility of a hematology instrument, the method comprising the steps of: a) providing a hematology control composition disclosed herein, wherein the hematology control composition has a known reference value for one or more blood cell parameters present in the hematology control composition; b) determining an experimental value for the one or more blood cell parameters using a hematology instrument; and c) comparing the experimental value for the one or more blood cell parameters obtained from step (b) with the known reference value for the one or more blood cell parameters from step (a), wherein the comparison determines the operational accuracy and reproducibility of the hematology instrument.

### BRIEF DESCRIPTION OF THE DRAWINGS

Figure 1 illustrates effects of free fatty acids on MCV stability.
Figure 2 illustrates effects of BSA monomer to dimer ratio on MCV stability.
Figure 3 illustrates MCV recovery in Formulation A, Lot A of five-part differential hematology cell control (Abnormal II) on a COULTER^{®} LH 750 instrument.
Figure 4 illustrates MCV recovery in Formulation A, Lot B of five-part differential hematology cell control (Abnormal II) on an LH 750 instrument.
Figure 5 illustrates MCV recovery in Formulation A, Lot C of five-part differential hematology cell control (Abnormal II) on an LH 750 instrument.
Figure 6 illustrates an MCV Recovery Comparison between one lot each of Formulation A and Formulation B on an LH 750 Instrument.
Figure 7 illustrates an MCV Recovery Comparison among all the Formulations on the LH 750 Instrument (2 additional lots of Formulation A, 1 additional lot of Formulation B, and 2 lots of Formulation C).
Figure 8 illustrates an MCV Recovery Comparison among all the Formulations on a COULTER^{®} STKS™ Instrument (2 lots of Formulation A, 1 lot of Formulation B, and 2 lots of Formulation C).
Figure 9 illustrates WBC Recovery of 3 lots of 5-part Differential Hematology Cell Control (Abnormal II) on an LH 750 Instrument.
Figure 10 illustrates RBC Recovery of 3 lots of 5-part Differential Hematology Cell Control (Abnormal II) on an LH 750 Instrument.
Figure 11 illustrates RDW Recovery of 3 lots of 5-part Differential Hematology Cell Control (Abnormal II) on an LH 750 Instrument.
Figure 12 illustrates Hemoglobin Recovery of 3 lots of 5-part Differential Hematology Cell Control (Abnormal II) on an LH 750 Instrument.
Figure 13 illustrates Platelet Recovery of 3 lots of 5-part Differential Hematology Cell Control (Abnormal II) on an LH 750 Instrument.
Figure 14 illustrates MPV Recovery of 3 lots of 5-part Differential Hematology Cell Control (Abnormal II) on an LH 750 Instrument.

### DETAILED DESCRIPTION

Described herein are suspension media compositions, hematology control compositions and methods for their use. A suspension media composition disclosed herein can be used with a blood cell component comprising one or more blood cells or their analogs. As such, a suspension media composition disclosed herein is suitable for the preparation of a hematology control composition. A suspension media composition disclosed herein can provide an extended stability for one or more blood cell components, and thus have a particular utility in preserving the shelf life of a hematology control composition over a longer period of time. In particular, the compositions can be useful for stabilizing the MCV and RDW of a red blood cell component of a hematology reference control, providing an extended shelf life. A longer shelf life may provide a manufacturer of a hematology control compositions with distribution channels for the compositions not previously available in products with shorter shelf lives. In addition, a longer shelf life may provide benefits to the end user by enabling the end user to purchase quantities of hematology compositions for subsequent use rather than purchasing a new supply of control products only after the existing supply has been exhausted or expired due to a shorter shelf life.

The hematology control compositions disclosed herein refer to suspension media compositions disclosed herein where one or more blood cell components comprising a red blood cell component are added. A blood cell component simulates a component found in whole blood and may or may not be fixed, stabilized, handled, treated, or prepared by other treatments prior to final suspension in a suspending media composition. A hematology control composition can be used in an multi-parameter automated hematology instrument and may be analyzed by electronic and optical measurements that enumerate and/or differentiate blood cells and their components. When measured by an instrument, a hematology control composition disclosed herein can assist in the calibration, operation, and accumulation of quality assurance data for the multi-parameter, automated hematology instrument.

The hematology control compositions disclosed herein comprise one or more serum albumin components. A serum albumin component, includes, without limitation, a bovine serum albumin or a human serum albumin purified from an organism or recombinants made.

In aspects of this embodiment, a hematology control composition comprises a serum albumin component present at a concentration of between, 30 g/L to 40 g/L, or 30 g/L to 50 g/L.

In other aspects of this embodiment, a hematology control composition comprises a serum albumin component present in a monomer to dimer ratio of, e.g., lower than 2:1, lower than 3:1, lower than 4:1, lower than 5:1, or lower than 6:1. In other aspects of this embodiment, a hematology control composition comprises a serum albumin component present in a monomer to dimer ratio of between, e.g., about 1:1 to about 2:1, about 1:1 to about 3:1, about 1:1 to about 4:1, about 1:1 to about 5:1 or about 1:1 to about 6:1.

The hematology control compositions disclosed herein comprise one or more cholesterol components.

In aspects of this embodiment, a hematology control composition comprises a cholesterol component present at a concentration of between, 400 mg/L to 1000 mg/L, 400 mg/L to 1100 mg/L, 400 mg/L to 1200 mg/L, 500 mg/L to 1000 mg/L, 500 mg/L to 1100 mg/L, 500 mg/L to 1200 mg/L, 600 mg/L to 1000 mg/L, 600 mg/L to 1100 mg/L, or 600 mg/L to 1200 mg/L.

The hematology control compositions disclosed herein may comprise one or more nonionic surfactant components. Non-limiting examples of nonionic surfactants include polyoxyethylene glycol sorbitan alkyl esters like polysorbate 20 sorbitan monooleate (TWEEN^{®} 20), polysorbate 40 sorbitan monooleate (TWEEN^{®} 40), polysorbate 60 sorbitan monooleate (TWEEN^{®} 60), polysorbate 61 sorbitan monooleate (TWEEN^{®} 61), polysorbate 65 sorbitan monooleate (TWEEN^{®} 65), polysorbate 80 sorbitan monooleate (TWEEN^{®} 80), and polysorbate 81 sorbitan monooleate (TWEEN^{®} 81); poloxamers (polyethylene-polypropylene copolymers), like Poloxamer 25R8 (PLURONIC^{®} 25R8), Poloxamer 124 (PLURONIC^{®} L44), Poloxamer 181 (PLURONIC^{®} L61), Poloxamer 182 (PLURONIC^{®} L62), Poloxamer 184 (PLURONIC^{®} L64), Poloxamer 188 (PLURONIC^{®} F68), Poloxamer 237 (PLURONIC^{®} F87), Poloxamer 338 (PLURONIC^{®} L108), Poloxamer 407 (PLURONIC^{®} F127); alkyl phenol polyglycol ethers; polyethylene glycol alkyl aryl ethers; polyoxyethylene glycol alkyl ethers, like octaethylene glycol monododecyl ether, pentaethylene glycol monododecyl ether, BRIJ^{®} 30, and BRIJ^{®} 35; 2-dodecoxyethanol (LUBROL^{®}-PX); polyoxyethylene glycol octylphenol ethers like polyoxyethylene (4-5) p-t-octyl phenol (TRITON^{®} X-45) and polyoxyethylene octyl phenyl ether (TRITON^{®} X-100); polyoxyethylene glycol alkylphenol ethers like Nonoxynol-9; phenoxypolyethoxylethanols like nonyl phenoxypolyethoxylethanol and octyl phenoxypolyethoxylethanol; glucoside alkyl ethers like octyl glucopyranoside; maltoside alkyl ethers like dodecyl maltopyranoside; thioglucoside alkyl ethers like heptyl thioglucopyranoside; digitonins; glycerol alkyl esters like glyceryl laurate; alkyl aryl polyether sulfates; alcohol sulfonates; sorbitan alkyl esters; cocamide ethanolamines like cocamide monoethanolamine and cocamide diethanolamine; sucrose monolaurate; dodecyl dimethylamine oxide, and sodium cholate. Other non-limiting examples of nonionic surfactants useful in the methods disclosed herein can be found in, *e.g.,* Winslow, et al., *Methods and Composition for Simultaneously Isolating Hemoglobin from Red Blood Cells and Inactivating Viruses,* U.S. 2008/0138790; Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003).

In aspects of this embodiment, a hematology control composition comprises a nonionic surfactant component present at a concentration of, *e.g.,* about 0.01% (v/v), about 0.05% (v/v), about 0.075% (v/v), about 0.1% (v/v), about 0.2% (v/v), about 0.3% (v/v), about 0.4% (v/v), about 0.5% (v/v), about 0.6% (v/v), about 0.7% (v/v), about 0.8% (v/v), about 0.9% (v/v), about 1.0% (v/v), about 2.0% (v/v), about 3.0% (v/v), about 4.0% (v/v), about 5.0% (v/v), about 6.0% (v/v), about 7.0% (v/v), about 8.0% (v/v), about 9.0% (v/v), or about 10.0% (v/v). In other aspects of this embodiment, a hematology control composition comprises a nonionic surfactant component present at a concentration of, *e.g.,* at least 0.01% (v/v), at least 0.05% (v/v), at least 0,075% (v/v), at least 0.1% (v/v), at least 0.25% (v/v), at least 0.5% (v/v), at least 0.75% (v/v), at least 1.0% (v/v), at least 2.5% (v/v), at least 5.0% (v/v), at least 7.5% (v/v), or at least 10.0% (v/v). In yet other aspects of this embodiment, a hematology control composition comprises a nonionic surfactant component present at a concentration of between, *e.g.,* about 0.1% (v/v) to about 0.5% (v/v), about 0.1% (v/v) to about 1.0% (v/v), about 0.2% (v/v) to about 0.5% (v/v), about 0.2% (v/v) to about 1.0% (v/v), about 0.2% (v/v) to about 2.0% (v/v),about 0.5% (v/v) to about 1.0% (v/v), about 0.5% (v/v) to about 5.0% (v/v), or about 1.0% (v/v) to about 10.0% (v/v).

The hematology control compositions disclosed herein may comprise one or more pH adjusting agents. Examples of suitable pH adjusting compositions which are suitable to impart the required pH include acids, typically citric, hydrochloric or sulfuric, and bases, such as alkali metal hydroxides and such other agents as known to those skilled in the art. Other acids may be substituted provided that they do not interfere with the analysis of the blood sample. Examples of acceptable pH adjusting agents include trisodium citrate and sodium phosphate. Examples of acceptable alkali metal hydroxides include sodium hydroxide and potassium hydroxide. In aspects of this embodiment, a hematology control composition comprises one or more pH adjusting agents providing a pH in the range of between, *e.g.,* about 5.8 to about 6.8, about 6.0 to about 6.8, about 6.2 to about 6.8, about 6:4 to about 6.8, about 5.8 to about 6.5, about 6.0 to about 6.5, or about 6.2 to about 6.5.

The hematology control compositions disclosed herein may comprise one or more osmolality agents. Suitable osmolality agents provide an osmolality in a range effective to maintain the red blood cells from lysis. The alkaline metal chloride and alkaline metal sulfate can be used to provide a suitable osmolality so as not to adversely affect the blood component in the hematology control product. Control of the osmolality concomitantly affects the red blood cell MCV. Suitable osmolality and tonicity adjusting agents include, without limitation, sodium chloride, potassium chloride, lactose, dextran and such other compounds as known to those skilled in the art. Generally, the suspension media may be iso-osmotic.

In aspects of this embodiment, a hematology control composition comprises one or more osmolality agents providing an osmolality of between, *e.g.,* about 200 mOsm to about 325 mOsm, about 200 mOsm to about 350 mOsm, about 200 mOsm to about 375 mOsm, about 200 mOsm to about 400 mOsm, about 250 mOsm to about 325 mOsm, about 250 mOsm to about 350 mOsm, about 250 mOsm to about 375 mOsm, about 250 mOsm to about 400 mOsm, about 280 mOsm to about 325 mOsm, about 280 mOsm to about 350 mOsm, about 280 mOsm to about 375 mOsm, or about 280 mOsm to about 400 mOsm. In an aspect of this embodiment, a hematology control composition comprises one or more osmolality agents providing an osmolality of between about 280 mOsm to about 330 mOsm.

The hematology control compositions disclosed herein may comprise one or more preservative agents. Suitable preservative agents provide fungicidal and bactericidal activity that reduce or prevent fungal or bacterial growth which might adversely affect the physical characteristics or functionality of the hematology control compositions disclosed herein. Suitable preservative agents may include sodium penicillin G, dihydrostreptomycin sulfate, 5-fluorouracil, imidazole, kanamycin sulfate, gentamycin sulfate and neomycin sulfate. However, the neomycin sulfate may affect the MCV of the red blood cells. The quantity of preservative agents should not adversely affect the analysis of the hematology control compositions disclosed herein.

In aspects of this embodiments, a hematology control composition comprises one or more preservative agents present in an amount of, *e.g.,* less than 0.5%, less than 0.4%, less than 0.3%, less than 0.2% by weight of the hematology control composition.

The hematology control compositions disclosed herein may comprise one or more supplementary agents. Suitable supplementary agents include, without limitation, dextran, ammonium chloride and dihydroxyacetone, dioctyphthalate, adenosine monophosphate and inosine.

The hematology control compositions disclosed herein may be substantially free of the unesterified fatty acid component.

In aspects of this embodiment, a hematology control composition comprises an unesterified fatty acid component having a concentration of, *e.g.,* less than 4 mmol/L, less than 3 mmol/L, less than 2mmol/L, less than 1.0 mmol/L, less than 0.75 mmol/L, less than 0.50 mmol/L, less than 0.25 mmol/L, less than 0.10 mmol/L, less than 0.075 mmol/L, less than 0.050 mmol/L, less than 0.025 mmol/L, less than 0.01 mmol/L, or less than 0.001 mmol/L In other aspects of this embodiment, a hematology control composition comprises an unesterified fatty acid component having a concentration of between, *e.g.,* about 0.01 mmol/L to about 4 mmol/L, about 0.01 mmol/L to about 3.5 mmol/L, about 0.01 mmol/L to about 3 mmol/L, about 0.01 mmol/L to about 2.5 mmol/L, about 0.01 mmol/L to about 2 mmol/L, about 0.1 mmol/L to about 4 mmol/L, about 0.1 mmol/L to about 3.5 mmol/L, about 0.1 mmol/L to about 3 mmol/L, about 0.1 mmol/L to about 2.5 mmol/L, or about 0.1 mmol/L to about 2 mmol/L. In another aspect of this embodiment, a hematology control composition comprises an unesterified fatty acid component having a concentration of less than about 0.78 mmol/L. In another aspect of this embodiment, a hematology control composition comprises an unesterified fatty acid component having a concentration of between about 0.78 mmol/L and about 2.16 mmol/L.

A blood cell component includes blood constituents, such as, *e.g.,* red blood cells, white blood cells, reticulocytes, platelets and nucleated red blood cells, etc, as well as analogs of there constituents. Preferably, a blood cell component comprises blood cells of human origin. In addition, a blood cell component may be derived from a source that will exhibit the size, shape or other measurable characteristic of human, animal, or other whole blood cell components as known in the art.

A hematology control composition comprises a red blood cell component. Blood cell components, alone or in combination with other blood cell components, sufficiently simulate the characteristics of whole blood which can be measured on an instrument, such as, *e.g.,* a hematological instrument. As such, blood cell components are useful in measurements of blood cell parameters, including, without limitation, blood cell size, blood cell shape, blood cell concentration, blood cell agglomeration, blood cell distribution, blood cell content, blood cell volume, blood cell population, blood cell type subpopulation, or any combination thereof.

In one embodiment, a hematology control composition may comprise a red blood component providing a red blood cell parameter. In aspects of this embodiment, the red blood cell component is a stabilized red blood cell component or a lyseable red blood cell component. In other aspects of this embodiment, the red blood cell parameter comprises red blood cell count, MCV, RDW, or any combination thereof. In other aspects of this embodiment, a hematology control composition comprises red blood cell count, MCV, and/or RDW which corresponds substantially with the red blood cell count, MCV, and/or RDW of human whole blood.

In another embodiment, a hematology control composition may comprise one or more white blood cell components. In aspects of this embodiment, the comprise one or more white blood cell components can simulate at least three subpopulations of white blood cells, at least four subpopulations of white blood cells, at least five subpopulations of white blood cells, at least six subpopulations of white blood cells, or at least seven subpopulations of white blood cells. In other aspects of this embodiment, a hematology control composition comprises one or more white blood cell subpopulations which correspond substantially with one or more white blood cell subpopulations of human whole blood.

In yet another embodiment, a hematology control composition may comprise one or more other blood components to resemble corresponding components in whole blood such as reticulocytes, platelets and nucleated red blood cells. In other aspects of this embodiment, a hematology control composition comprises one or more blood components which correspond substantially with reticulocytes, platelets and/or nucleated red blood cells of human whole blood.

The hematology control compositions disclosed herein provide a stable shelf life of one or more blood components. A stable shelf life of a blood component refers to a hematology control composition comprising a blood component that is still useful in the calibration, operation, and/or accumulation of quality assurance data for a hematology instrument. In other words, the one or more blood cell components are still stable enough to obtain one or more useful measurements from a hematology instrument. The stability of a hematology control composition and/or blood cell component can be measured by a change over time in a reference value representative of the shelf life of the composition. A reference value representative of the stability or shelf life of the composition can in some embodiments be a mean cell volume value, such as a MCV, or in addition or in the alternative, a RDW value.

In an aspect of this embodiment, a hematology control composition comprising a blood component has a stable shelf life of the blood component when one or more measurements obtained for calibration, operation, and/or accumulation of quality assurance data from a hematology instrument using a hematology control composition that was prepared at least, 30 days earlier are substantially the same measurements obtained using a similar hematology control composition, but freshly prepared. In another aspect of this embodiment, a hematology control composition comprising a blood component has a stable shelf life of the blood component when one or more measurements obtained for calibration, operation, and/or accumulation of quality assurance data from a hematology instrument using a hematology control composition that was prepared at least 60 days earlier are substantially the same measurements obtained using a similar hematology control composition, but freshly prepared. In yet another aspect of this embodiment, a hematology control composition comprising a blood component has a stable shelf life of the blood component when one or more measurements obtained for calibration, operation, and/or accumulation of quality assurance data from a hematology instrument using a hematology control composition that was prepared at least 95 days earlier are substantially the same measurements obtained using a similar hematology control composition, but freshly prepared. In still another aspect of this embodiment, a hematology control composition comprising a blood component has a stable shelf life of the blood component when one or more measurements obtained for calibration, operation, and/or accumulation of quality assurance data from a hematology instrument using a hematology control composition that was prepared at least 30 days earlier are substantially the same measurements obtained using a similar hematology control composition, but freshly prepared.

In another aspect of this embodiment, a hematology control composition comprising a blood component has a stable shelf life of the blood component when one or more measurements obtained for calibration, operation, and/or accumulation of quality assurance data from a hematology instrument using a hematology control composition that was prepared about 30 days to about 95 days earlier are substantially the same measurements obtained using a similar hematology control composition, but freshly prepared. In yet another aspect of this embodiment, a hematology control composition comprising a blood component has a stable shelf life of the blood component when one or more measurements obtained for calibration, operation, and/or accumulation of quality assurance data from a hematology instrument using a hematology control composition that was prepared about 95 days to about 175 days earlier are substantially the same measurements obtained using a similar hematology control composition, but freshly prepared.

The hematology control compositions disclosed herein may comprise a serum albumin component and a cholesterol component present in concentrations sufficient to provide a stable shelf life of a blood component. A stable shelf life of a blood component refers to a hematology control composition comprising a blood component that when used in a hematology instrument will assist in the calibration, operation, and/or accumulation of quality assurance data for the hematology instrument.

In other aspects of this embodiment, a hematology control composition comprises a serum albumin component and a cholesterol component present in concentrations sufficient to provide a stable shelf life of a blood component of, *e.g.,* at least 30 days, at least 40 days, at least 50 days, at least 60 days, at least 70 days, at least 80 days, at least 90 days, at least 100 days, at least 110 days, at least 120 days, at least 130 days, at least 140 days, at least 150 days, at least 160 days, at least 170 days, at least 180 days, at least 190 days, or at least 200 days. In aspects of this embodiment, a hematology control composition comprises a serum albumin component and a cholesterol component present in concentrations sufficient to provide a stable shelf life of a blood component of between, *e.g.,* about 40 days to about 180 days, about 50 days to about 180 days, about 60 days to about 180 days, about 70 days to about 180 days, about 80 days to about 180 days, about 90 days to about 180 days, about 100 days to about 180 days, about 40 days to about 200 days, about 50 days to about 200 days, about 60 days to about 200 days, about 70 days to about 200 days, about 80 days to about 200 days, about 90 days to about 200 days, or about 100 days to about 200 days. In an aspect of this embodiment, a hematology control composition comprises a serum albumin component and a cholesterol component present in concentrations sufficient to provide a stable shelf life of a blood component of between 95 days and 175 days.

Aspects of the present specification also disclose methods to determine operational accuracy and reproducibility of a hematology instrument. The methods comprise the steps of: a) providing a hematology control composition disclosed herein, wherein the hematology control composition has a known reference value for one or more blood cell parameters present in the composition; b) determining an experimental value for the one or more blood cell parameters using a hematology instrument; and c) comparing the experimental value of the one or more blood cell parameters obtained from step (b) with the known reference value of the one or more blood cell parameters from step (a), wherein the comparison is determinative of operational accuracy and reproducibility of the hematology instrument. Measurements of the known reference value for the one or more blood cell parameters and the experimental value for the one or more blood cell parameters can be made using light scatter, low frequency current, radio frequency current, fluorescence, absorbance, and combinations thereof.

Step (a) refers to a known reference value for one or more blood cell parameters in the hematology control composition. A known reference value refers to a value of a blood cell parameter determined when a blood cell component is first mixed with a suspension media composition disclosed herein. A known reference value may be a value of a blood cell parameter determined for the same hematology control composition subsequently used or may be an averaged value of a blood cell parameter determined from multiple hematology control compositions determined at the same or different times. Blood cell parameters, including, without limitation, blood cell size, blood cell shape, blood cell concentration, blood cell agglomeration, blood cell distribution, blood cell content, blood cell volume, blood cell population, blood cell type subpopulation, or any combination thereof. A known reference value of a blood cell parameter is the standard for which all subsequent values will be compared.

In aspects of this embodiment, a reference value for one or more blood cell parameters comprises a MCV value, a RDW value, or both. In other aspects of this embodiment, a reference value for one or more blood cell parameters comprises one or more white blood cell subpopulations.

Step (b) refers to an experimental value for one or more blood cell parameters in the hematology control composition. An experimental value refers to a value of a blood cell parameter determined when a blood cell component is subsequently used after its initial preparation with a suspension media composition. The subsequent use can be any length of time from the initial preparation of the hematology control composition. Blood cell parameters, including, without limitation, blood cell size, blood cell shape, blood cell concentration, blood cell agglomeration, blood cell distribution, blood cell content, blood cell volume, blood cell population, blood cell type subpopulation, or any combination thereof. An experimental value of a blood cell parameter is one that is subsequently determined and compared to a known reference value for the blood cell parameter.

In aspects of this embodiment, an experimental value for one or more blood cell parameters comprises a MCV value, a RDW value, or both. In other aspects of this embodiment, an experimental value for one or more blood cell parameters comprises one or more white blood cell subpopulations.

In aspects of this embodiment, a subsequent use of a hematology control composition may be, *e.g.,* at least 30 days, at least 40 days, at least 50 days, at least 60 days, at least 70 days, at least 80 days, at least 90 days, at least 100 days, at least 110 days, at least 120 days, at least 130 days, at least 140 days, at least 150 days, at least 160 days, at least 170 days, at least 180 days, at least 190 days, or at least 200 days, after the initial preparation of the hematology control composition. In aspects of this embodiment, a subsequent use of a hematology control composition may be between, *e.g.,* about 40 days to about 180 days, about 50 days to about 180 days, about 60 days to about 180 days, about 70 days to about 180 days, about 80 days to about 180 days, about 90 days to about 180 days, about 100 days to about 180 days, about 40 days to about 200 days, about 50 days to about 200 days, about 60 days to about 200 days, about 70 days to about 200 days, about 80 days to about 200 days, about 90 days to about 200 days, or about 100 days to about 200 days, after the initial preparation of the hematology control composition. In an aspect of this embodiment, a subsequent use of a hematology control composition may be between 30 days and 95 days after the initial preparation of the hematology control composition. In an aspect of this embodiment, a subsequent use of a hematology control composition may be between 95 days and 175 days after the initial preparation of the hematology control composition.

Step (c) refers to a comparison of an experimental value of one or more blood cell parameters to a known reference value for the same one or more blood cell parameters. This comparison step determines the accuracy and reproducibility of the operation of the hematology instrument. In aspects of this embodiment, an experimental value of the one the one or more blood cell parameter that is at least 75%, at least 80%, at least 85%, at least 90%, at least 93%, at least 95%, or at least 97% of a known reference value for the same one or more blood cell parameters is indicative of an accurate and reproducible operation of the hematology instrument.

In another aspect of this embodiment, an experimental value of the one the one or more blood cell parameter that is between about 75% to about 100% of a known reference value for the same one or more blood cell parameters is indicative of an accurate and reproducible operation of the hematology instrument. In yet another aspect of this embodiment, an experimental value of the one the one or more blood cell parameter that is between about 85% to about 100% of a known reference value for the same one or more blood cell parameters is indicative of an accurate and reproducible operation of the hematology instrument. In still another aspect of this embodiment, an experimental value of the one the one or more blood cell parameter that is between about 90% to about 100% of a known reference value for the same one or more blood cell parameters is indicative of an accurate and reproducible operation of the hematology instrument. In another aspect of this embodiment, an experimental value of the one the one or more blood cell parameter that is between about 95% to about 100% of a known reference value for the same one or more blood cell parameters is indicative of an accurate and reproducible operation of the hematology instrument. In yet another aspect of this embodiment, an experimental value of the one the one or more blood cell parameter that is between about 97% to about 100% of a known reference value for the same one or more blood cell parameters is indicative of an accurate and reproducible operation of the hematology instrument.

### EXAMPLES

### Example 1

### Suspension Media Composition Formulation and Hematological Control Composition Preparation

### Free fatty acid component

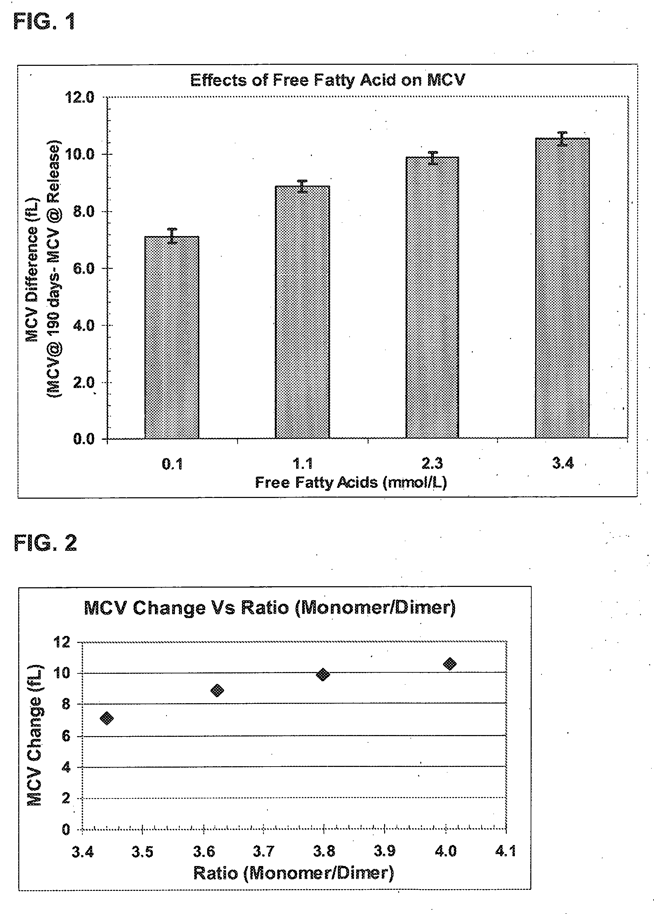

An experimental design was performed to determine the effects of free fatty acid on MCV change using the same mixture of a red blood cell pool. Table 1 shows the ranges for each component of the suspension media composition. A hematological control composition was prepared by adding red blood cells to the formulation in a concentration of approximately 1.8 × 10⁶ RBC/microliter, which is known as Abnormal II. Figure 1 shows the strong relationship between MCV change and free fatty acid levels. MCV change increases with the increase of fatty acid level in the suspension media.

| **Table 1: Components of Suspension Media** | |
|---|---|
| **Components** | **Approximate Amounts in Liter Formulation** |
| 1. Sodium Chloride | 2 g |
| 2. Sodium Phosphate Monobasic | 0.8 to 2.5 g |
| 3. Methlyparaben-7m | 0.5 to 1.0 g |
| 4. Propyl Paraben | 0.3 to 1.0 g |
| 5. Lactose | 10 to 50 g |
| 6. Citric Acid (Rgt.Anhydrous) | 0.3 to 0.9 g |
| 7. Neomycin Sulfate | 0.2 to 1.0 g |
| 8. Adenosines'-Monophosphate (AMP) | 0.4 to 1.0 g |
| 9. Sodium Hydroxide | 0.46 g |
| 10. Tri-Sodium Citrate | 3.0 to 8.0 g |
| 11. Deoxycholic Acid | 0.1 to 0.9 g |
| 12. Sodium Penicillin G | 0.5 x 10⁶ to 3 x 10⁶ units |
| 13. Kanamycin Sulfate | 0.2 to 0.8 g |
| 14. Inosine | 0.4 to 1.0 g |
| 15. Dihydrostreptomycin Sulfate | 0.2 to 1.0 g |
| 16. Procaine HCl | 0.1 to 0.5 g |
| 17. lodoacetamide | 0.037 g |
| 18. Phenergan HCl | 0.1 to 1.0 g |
| 19. 5-Fluorouracil | 1 g |
| 20. Pluronic 25R8 | 2 g |
| 21. BSA Solution | 30 to 50 g |
| 22. Cholesterol | 400 to 1200 mg |
| 23. q.s. to 1 L with Deionized Water | |

### BSA dimer and monomer components

BSA interacts with the phospholipid membrane of red blood cells (RBCs) and makes a coating on the RBC surface by electrostatic interaction, preventing lysis of RBCs. Also, BSA may exist in a monomer or dimer form, and that the process of removing free fatty acid during manufacturing of reagent grade BSA causes formation of BSA dimer. The effect of BSA monomer and dimer on MCV change in RBCs was studied. The percentages of BSA monomer and BSA dimer present in the suspension media were obtained using high performance liquid chromatography (HPLC). Figure 2 shows the effects of BSA monomer to dimer ratios on MCV stability. A lower BSA monomer to dimer ratio, in addition to the suspension media being substantially fatty acid free, provides longer MCV stability.

### Example 2

### Hematological Control Composition Stability Testing

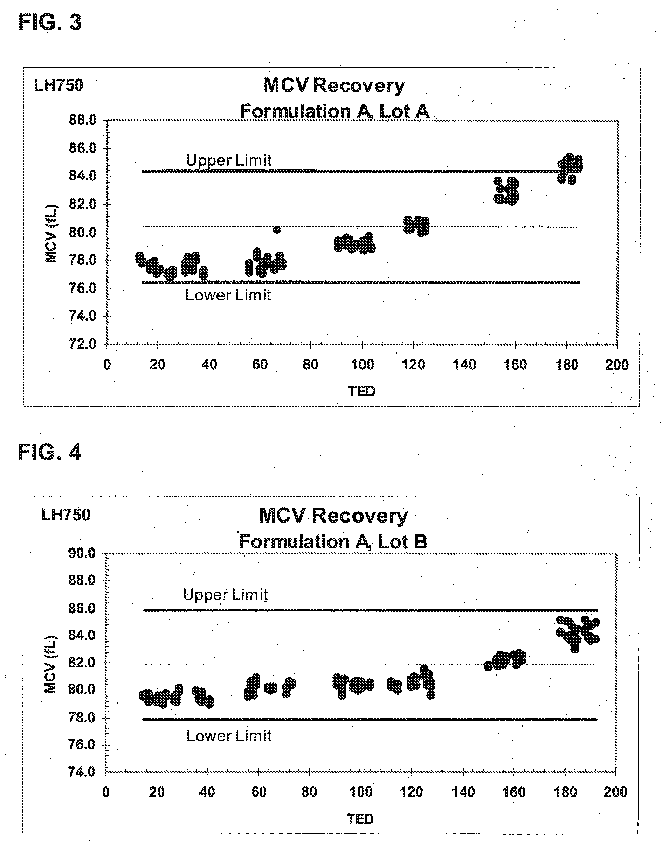

A suspension medium referred to herein as Formulation A was made using reagent grade BSA substantially free of unesterified fatty acids, and including RBC levels and other components as provided in Example 1. The free fatty acid level of Formulation A is provided in Table 2. Figures 3-5 show the MCV recovery of five-part differential hematology cell controls with low RBC levels known as Abnormal II, measured on an LH 750 instrument. All the lots of suspension media showed a minimum MCV stability of 175 days for Formulation A.

| **Table 2: Suspension Media Formulation** | | |
|---|---|---|
| **Suspension Media** | **Components** | **Free Fatty Acid Contents (mmol/L)** |
| Formulation A | Table 1 | < 0.78 |
| Formulation B | Table 1 | >3.4 |
| Formulation C | Table 1 | 0.78 to 2.16 |

### Example 3

### Hematological Control Composition Stability Testing

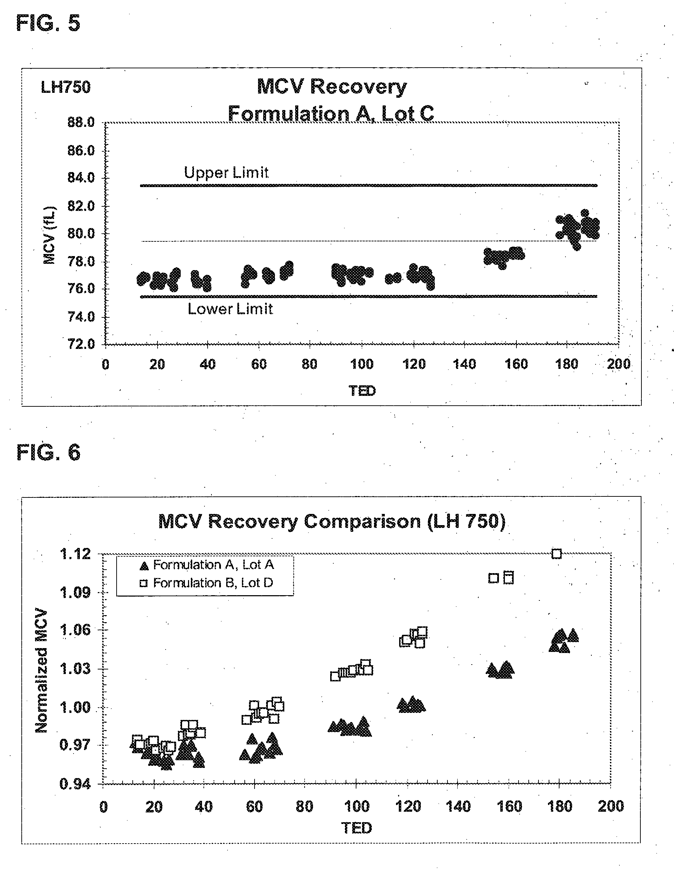

A suspension medium referred to herein as Formulation B was made using standard grade BSA without removing free fatty acids. Other components including RBC levels were kept the same as provided in Example 1. The free fatty acid level of Formulation B is also provided in Table 2. The MCV recovery comparison plots of one lot each of Formulation A and Formulation B measured on an LH 750 Instrument are provided in Figure 6. Normalized data were obtained from the mean values of each lot. As Figure 6 shows, the MCV change of Formulation A is much lower than that of Formulation B.

### Example 4

### Hematological Control Composition Stability Testing

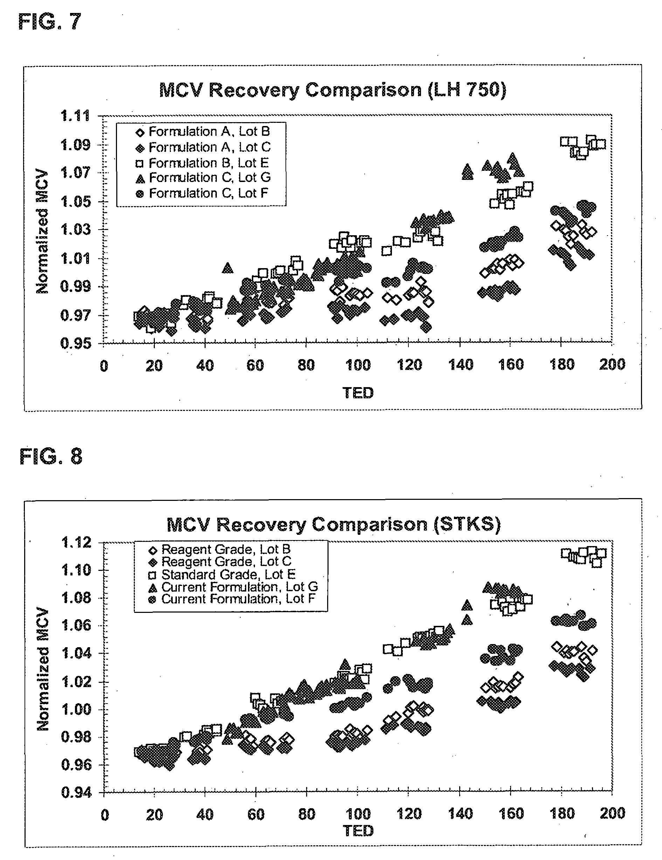

A suspension medium referred to herein as Formulation C was made using MOD-U-CYTE^{®} (Miles Laboratories, Inc, Elkhart, IN) VIII Media (see US Patent 5,529,933), with the free fatty acids level shown in Table 2. Components of the suspension media including RBC levels were kept the same as provided in Example 1. MCV recovery comparison plots of three different formulations (Formulation A, Formulation B, and Formulation C) measured on LH 750 and STKS instruments are provided in Figures 7 and 8, respectively. Data were normalized using the mean values of each lot.

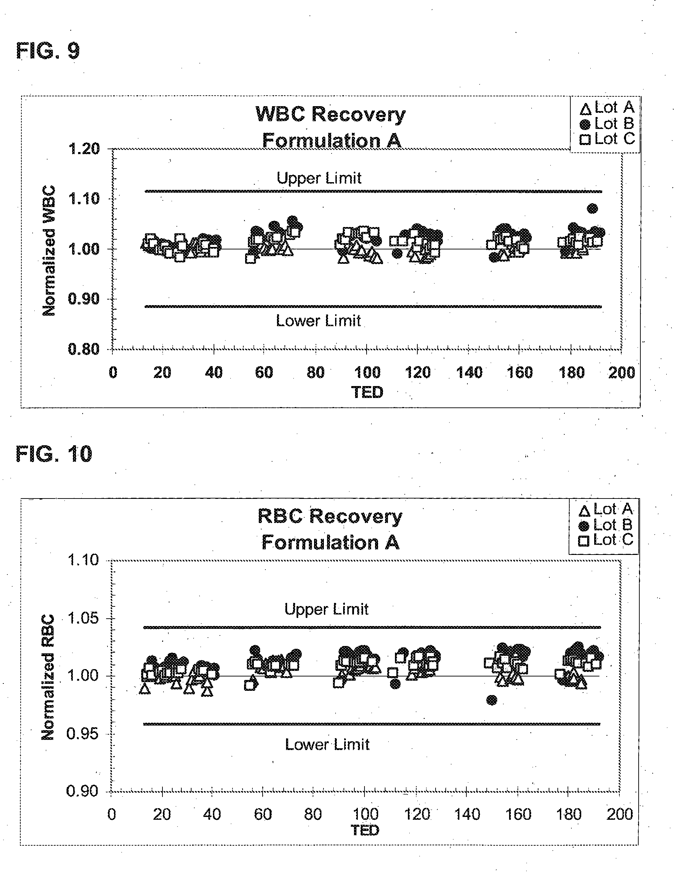

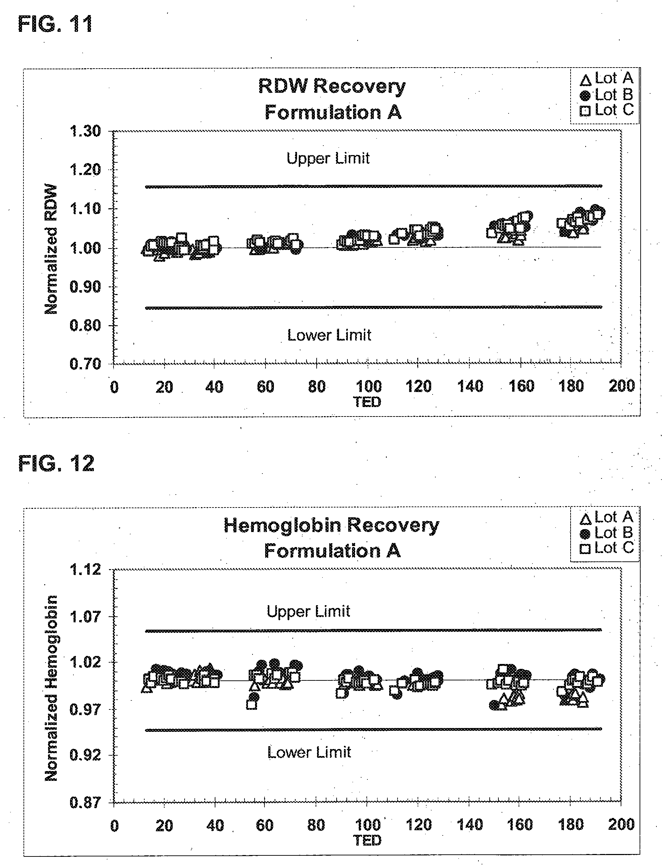

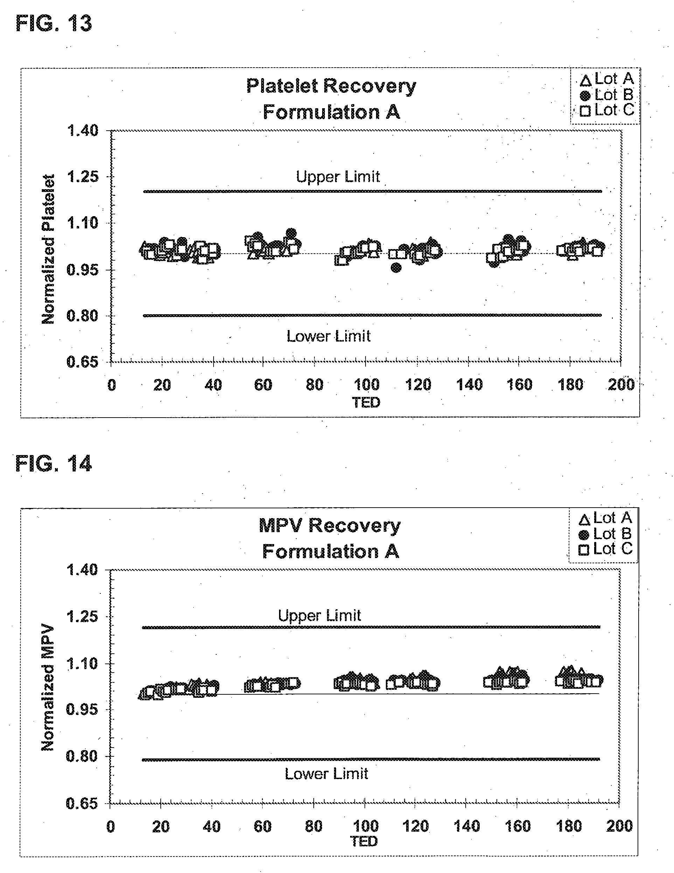

All the comparison plots of data measured on both the instruments showed that the MCV changes using Formulation A as the suspension medium are the lowest, thus Formulation A showed the greatest MCV stability. Figures 9 to 14 illustrate the stability of various blood cell parameters (WBC, RBC, RDW, Hemoglobin, Platelet, and MPV) using Formulation A measured on an LH 750 instrument. There were no significant changes to all of these parameters, and their recovery values were near the release assay value. Components for 5-part differential cell control suspension media were qualified to provide improved suspension media (longer stability and shelf life) than media previously available. It is herein shown that free fatty acid, cholesterol and BSA component amounts within the suspension medium have important effects on the blood mean cell volume (MCV) stability in the Cell Control products, especially in the lower level of RBC (i.e. 5-part differential Cell Control - Abnormal II). Evaluation of three lots of 5-part differential Cell Control Abnormal (II) product made with improved suspension media (i.e. suspension media prepared using BCS raw material with Reagent Grade of BSA or Fatty Acid free BSA) showed the longest stability of MCV values compared to the other two raw materials (BSA with fatty acid and MOD-U-CYTE^{®} VIII Media). The compositions and methods herein provide unique suspension media for hematology cell controls to be used for 5 part differential counts with a longer stability and improved shelf life.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein.

The terms "a," "an," "the" and similar referents used in the context of describing the present invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the present invention so claimed are inherently or expressly described and enabled herein.

## Claims

1. A hematology control composition comprising a red blood cell component and an isotonic suspension medium including a serum albumin component and a cholesterol component, wherein the composition has a free fatty acid concentration of less than 4 mmol/liter; and wherein the serum albumin component is present at a concentration of from 30 grams/liter to 50 grams/liter; and wherein the serum albumin component comprises a ratio of serum albumin monomer to serum albumin dimer lower than 6:1; and wherein the cholesterol component is present at a concentration of from 400 mg/liter to 1200 mg/liter.

2. The composition of claim 1, wherein the serum albumin component and the cholesterol component are present in amounts sufficient to provide a shelf life of the composition of at least ninety-five days.

3. The composition of claim 2, wherein the shelf life of the composition is represented by a stabilization in mean cell volume, a stabilization in red blood cell distribution width, or both.

4. The composition of claim 1, wherein the red blood cell component is present in an amount sufficient to be measurable with an automated hematology instrument.

5. The composition of claim 1, wherein the serum albumin component comprises a ratio of serum albumin monomer to serum albumin dimer lower than 5:1.

6. The composition of claim 1, further comprising a nonionic surfactant component.

7. The composition of claim 6, wherein the nonionic surfactant component comprises a poloxamer.

8. The composition of claim 1, which further comprises a white blood cell component.

9. The composition of claim 8, wherein the white blood cell component simulates at least three sub populations of white blood cells.

10. The composition of claim 9, wherein the white blood cell component simulates five subpopulations of white blood cells.

11. The composition of claim 1, which further comprises a compatible preservative component in an amount sufficient to provide fungicidal and bactericidal activity.

12. The hematology control composition of claim 1, which further comprises one or more pH adjusting agents to provide a pH in the range from 5.8 to 6.8.

13. A method to determine operational accuracy and reproducibility of a hematology instrument, the method comprising the steps of:
a) providing a hematology control composition according to any one of claims 1 to 12, wherein the composition has a known reference value for one or more blood cell parameters present in the hematology control composition;
b) determining an experimental value for the one or more blood cell parameters with a hematology instrument; and
c) comparing the experimental value for the one or more blood cell parameters obtained from step (b) with the known reference value for the one or more blood cell parameters from step (a), wherein the comparison determines the operational accuracy and reproducibility of the hematology instrument.

## Patentansprüche

1. Hämatologiekontrollzusammensetzung, umfassend eine rote Blutzellkomponente und ein isotonisches Suspensionsmedium, umfassend eine Serumalbumin-Komponente und eine Cholesterin-Komponente, wobei die Zusammensetzung eine Konzentration an freier Fettsäure von weniger als 4 mmol/Liter aufweist; und wobei die Serumalbumin-Komponente in einer Konzentration von 30 Gramm/Liter bis 50 Gramm/Liter vorhanden ist; und wobei die Serumalbumin-Komponente ein Verhältnis von Serumalbumin-Monomer zu Serumalbumin-Dimer von weniger als 6:1 umfasst; und wobei die Cholesterin-Komponente in einer Konzentration von 400 mg/Liter bis 1200 mg/Liter vorhanden ist.

2. Zusammensetzung nach Anspruch 1, wobei die Serumalbumin-Komponente und die Cholesterin-Komponente in Mengen vorhanden sind, die ausreichen, um eine Haltbarkeitsdauer der Zusammensetzung von mindestens fünfundneunzig Tagen bereitzustellen.

3. Zusammensetzung nach Anspruch 2, wobei die Haltbarkeitsdauer der Zusammensetzung durch eine Stabilisierung des mittleren Zellvolumens, eine Stabilisierung der Verteilungsbreite roter Blutzellen oder beides dargestellt wird.

4. Zusammensetzung nach Anspruch 1, wobei die rote Blutzellkomponente in einer Menge vorhanden ist, die ausreicht, um mit einem automatisierten Hämatologieinstrument messbar zu sein.

5. Zusammensetzung nach Anspruch 1, wobei die Serumalbumin-Komponente ein Verhältnis von Serumalbumin-Monomer zu Serumalbumin-Dimer von weniger als 5:1 umfasst.

6. Zusammensetzung nach Anspruch 1, ferner umfassend eine nichtionische Tensidkomponente.

7. Zusammensetzung nach Anspruch 6, wobei die nichtionische Tensidkomponente ein Poloxamer umfasst.

8. Zusammensetzung nach Anspruch 1, die ferner eine weiße Blutzellkomponente umfasst.

9. Zusammensetzung nach Anspruch 8, wobei die weiße Blutzellkomponente mindestens drei Subpopulationen von weißen Blutzellen simuliert.

10. Zusammensetzung nach Anspruch 9, wobei die weiße Blutzellkomponente fünf Subpopulationen von weißen Blutzellen simuliert.

11. Zusammensetzung nach Anspruch 1, die ferner eine kompatible Konservierungskomponente in einer Menge umfasst, die ausreicht, um eine fungizide und bakterizide Wirkung bereitzustellen.

12. Hämatologiekontrollzusammensetzung nach Anspruch 1, die ferner einen oder mehrere pH-Wert-Regler umfasst, um einen pH-Wert im Bereich von 5,8 bis 6,8 bereitzustellen.

13. Verfahren zur Ermittlung der Betriebsgenauigkeit und Reproduzierbarkeit eines Hämatologieinstruments, wobei das Verfahren die Schritte umfasst:
a) Bereitstellen einer Hämatologiekontrollzusammensetzung nach einem der Ansprüche 1 bis 12, wobei die Zusammensetzung einen bekannten Referenzwert für einen oder mehrere in der Hämatologiekontrollzusammensetzung vorhandene Blutzellparameter aufweist;
b) Ermitteln eines experimentellen Werts für den einen oder die mehreren Blutzellparameter mit einem Hämatologieinstrument; und
c) Vergleichen des aus Schritt (b) erhaltenen experimentellen Werts für den einen oder die mehreren Blutzellparameter mit dem bekannten Referenzwert für den einen oder die mehreren Blutzellparameter aus Schritt (a), wobei der Vergleich die Betriebsgenauigkeit und Reproduzierbarkeit des Hämatologieinstruments bestimmt.

## Revendications

1. Composition de contrôle hématologique comprenant un composant de globule rouge et un milieu de suspension isotonique comprenant un composant d'albumine sérique et un composant de cholestérol, où la composition a une concentration en acides gras libres inférieure à 4 mmol/litre ; et dans laquelle le composant d'albumine sérique est présent à une concentration allant de 30 grammes/litre à 50 grammes/litre ; et dans laquelle le composant d'albumine sérique comprend un rapport du monomère d'albumine sérique au dimère d'albumine sérique inférieur à 6:1 ; et dans laquelle le composant de cholestérol est présent à une concentration allant de 400 mg/litre à 1200 mg/litre.

2. Composition selon la revendication 1, dans laquelle le composant d'albumine sérique et le composant de cholestérol sont présents en quantités suffisantes pour fournir une durée de conservation de la composition d'au moins quatre-vingt-quinze jours.

3. Composition selon la revendication 2, dans laquelle la durée de conservation de la composition est représentée par une stabilisation en volume cellulaire moyen, une stabilisation de la largeur de distribution des globules rouges ou l'une et l'autre.

4. Composition selon la revendication 1, dans laquelle le composant de globules rouges est présent en une quantité suffisante pour être mesurable avec un instrument hématologique automatisé.

5. Composition selon la revendication 1, dans laquelle le composant d'albumine sérique comprend un rapport de monomère d'albumine sérique à dimère d'albumine sérique inférieur à 5:1.

6. Composition selon la revendication 1, comprenant en outre un composant tensioactif non ionique.

7. Composition selon la revendication 6, dans laquelle le composant tensioactif non ionique comprend un poloxamère.

8. Composition selon la revendication 1, qui comprend en outre un composant de globules blancs.

9. Composition selon la revendication 8, dans laquelle le composant de globules blancs simule au moins trois sous-populations de globules blancs.

10. Composition selon la revendication 9, dans laquelle le composant de globules blancs simule cinq sous-populations de globules blancs.

11. Composition selon la revendication 1, qui comprend en outre un composant conservateur compatible en quantité suffisante pour fournir une activité fongicide et bactéricide.

12. Composition de contrôle hématologique selon la revendication 1, qui comprend en outre un ou plusieurs agents d'ajustement de pH pour fournir un pH compris entre 5,8 et 6,8.

13. Procédé pour déterminer la précision de fonctionnement et la reproductibilité d'un instrument hématologique, le procédé comprenant les étapes consistant à :
a) fournir une composition de contrôle hématologique selon l'une quelconque des revendications 1 à 12, dans lequel la composition a une valeur de référence connue pour un ou plusieurs paramètres de cellules sanguines présents dans la composition de contrôle hématologique ;
b) déterminer une valeur expérimentale pour le ou les paramètres de cellules sanguines avec un instrument d'hématologie ; et
c) comparer la valeur expérimentale pour le ou les paramètres de cellules sanguines, obtenue à l'issue de l'étape (b), avec la valeur de référence connue pour le ou les paramètres de cellules sanguines de l'étape (a), dans lequel la comparaison détermine la précision de fonctionnement et la reproductibilité de l'instrument hématologique.
